# EUROPÄISCHE PATENTSCHRIFT

(11) **EP 2 229 120 B1**
(45) Veröffentlichungstag und Bekanntmachung des Hinweises auf die Patenterteilung: **23.02.2011**
(21) Anmeldenummer: 08862915.9
(22) Anmeldetag: 17.12.2008
(51) Int. Cl.: A61B 19/02, G01K 1/08

(54) **FOLIENSCHLAUCHABDECKUNG FÜR MEDIZINISCHE GERÄTE**
TUBULAR FILM COVER FOR MEDICAL DEVICES
REVETEMENT DE FILMS TUBULAIRES POUR APPAREILS MEDICAUX

(30) Priorität: 19.12.2007 DE 102007061805
(43) Veröffentlichungstag der Anmeldung: 22.09.2010
(73) Patentinhaber: Udo Heisig GmbH, 85640 Putzbrunn (DE)
(72) Erfinder: HEISIG, Udo, 85640 Putzbrunn (DE)
(74) Vertreter: Feldkamp, Rainer
(86) Internationale Anmeldenummer: PCT/EP2008/010778
(87) Internationale Veröffentlichungsnummer: WO 2009/077176

(56) Entgegenhaltungen:
- EP-A1- 1 619 527
- DE-U1- 8 812 027
- DE-U1- 9 012 429
- DE-U1- 9 418 340

## Beschreibung

Die Erfindung bezieht sich auf eine Folienschlauchabdeckung für medizinische Ultraschallgeräte der im Oberbegriff des Anspruchs 1 genannten Art.

Bei vielen Geräten im medizinischen Bereich, beispielsweise bei Ultraschallgeräten, sind diese Geräte mit Steuer- und Auswerteeinrichtungen über lang gestreckte Schläuche und/oder Kabel verbunden. Sowohl die Geräte als auch die Schläuche und/oder Kabel müssen mit sterilen Abdeckungen versehen werden. Zu diesem Zweck ist es bekannt, Folienschlauchabdeckungen zu verwenden, die vor dem Gebrauch mit einer sogenannten Steckfaltung auf eine sehr kurze Länge teleskopartig zusammengeschoben oder gefaltet sind. Das offene Ende der Folienschlauchabdeckung kann an einem Haltering befestigt sein, der gleichzeitig zur Aufnahme des in seiner Länge verkürzten Folienschlauches vor Gebrauch verwendet wird. Eine andere Möglichkeit besteht darin, die Steckfaltung in einer verstärkten Kartonhülse oder ähnlichem aufzubewahren, die gleichzeitig als Öffnungshilfe dient. Folienschlauchabdeckungen dieser Art sind in vielfältiger Art bekannt. Bei jeder dieser Möglichkeiten bleibt jedoch das Problem bestehen, dass das medizinische Gerät innerhalb der Folienschlauchabdeckung nicht fixiert ist. Es kann jederzeit aus der Folie herausgleiten, oder sich gegenüber dieser verschieben. Dies gilt insbesondere bei Ultraschallgeräten, bei denen zwischen der Kontaktfläche des Gerätes und dem Folienschlauch ein Gel aufgebracht wird, um die gleichförmige Signalübertragung zu ermöglichen.

Aus der DE 90 12 429 U1 ist eine Folienschlauchabdeckung für ein medizinisches Ultraschallgerät mit einem durch eine Steckfaltung verkürzten Folienschlauch bekannt, der einen offenen und einen geschlossenen Endabschnitt und eine sterile und eine nicht sterile Seite aufweist. Der geschlossene Endabschnitt bildet im gefalteten Zustand des Folienschlauchs auf der nicht sterilen Seite eine Art von Tasche, in die vor dem Einstecken des Ultraschallgerätes ein Ultraschall-Gel oder ein anderes Ankoppelmittel eingefüllt werden kann, um eine gute Kopplung des Ultraschallgerätes mit dem Folienschlauch und dessen steriler Seite zu erreichen. Die Verwendung von Ankoppelmitteln ist zeitaufwendig und erfordert eine spätere Reinigung des Ultraschallgerätes.

Weiterhin ist aus der EP 1619 527 A1 eine Schutzhülle für einen faseroptischen Katheter bekannt, die ein Ende aufweist, das durch ein optisches Fenster verschlossen ist. Das optische Fenster besteht aus einem festen Gel-Material, das bei einem Anpressen des Katheters eine Klebewirkung entfalten kann. Ein Ultraschallgerät weist zumeist eine größere aktive Fläche auf, als ein optischer Katheter, so dass abgesehen von der zu großen Dämpfung von UltraschallSchwingungen in dem Gel-Material eine ausreichende Klebekraft und eine gleichförmige Schallübertragung über die gesamte aktive Fläche des Ultraschallgerätes mit einem derartigen Gel-Material nicht erreicht werden könnte.

Der Erfindung liegt die Aufgabe zugrunde, eine Folienschlauchabdeckung für ein medizinisches Ultraschallgerät zu schaffen, die bei einfachem Aufbau eine verbesserte Handhabung und eine gute Schallübertragung ermöglicht.

Diese Aufgabe wird durch die im Patentanspruch 1 angegebenen Merkmale gelöst.

Es wird eine Folienschlauchabdeckung geschaffen, die an ihrem geschlossenen Endabschnitt eine Klebefläche in Form einer sehr dünnen zweiseitig klebenden

Klebefolie aufweist, an der das medizinische Gerät mit der Schallfläche befestigt wird. Diese Klebefolie dient ferner auch als Übertragungsmedium, so dass kein zusätzliches Gel zwischen Ultraschall-Kopf und Folienschlauch benötigt wird. Die Klebefolie ist derart ausgestaltet, dass bei vorhandenen Falten ein nachträgliches Ablösen der Klebefolie von dem Gerät und/oder ein Nachstraffen der Folie ermöglicht wird. Eine solche Klebefolie ist sowohl bei einer einfachen Steckfaltung als auch bei einem Folienschlauch, der in einem Haltering aufbewahrt wird, oder einer Steckfaltung, die eine Kartonhülse als Öffnungsvorrichtung aufweist, oder einem Beutel mit beliebiger Breite und Länge anwendbar.

Die Klebefolie befindet sich auf der nicht sterilen Seite der Folienschlauchabdeckung. Die Klebefolie weist an ihren Kanten bevorzugt Einkerbungen auf, durch die hindurch eine Faltenbildung des Folienschlauchs bei der Anwendung für medizinische Geräte mit gekrümmter Kontaktfläche ermöglicht wird, so dass eine glatte Anlage des Folienschlauchs erreicht wird.

Vorteilhafte Ausgestaltungen und Weiterbildungen der Erfindung ergeben sich aus den Unteransprüchen.

Eine Ausführungsform der Erfindung wird im Folgenden anhand von Zeichnungen näher erläutert.

In den Zeichnungen zeigen:
- Figur 1: eine Ansicht der Folienschlauchabdeckung,
- Figur 2: eine Schnittdarstellung der Folienschlauchabdeckung,
- Figur 3: eine Ansicht der Folienschlauchabdeckung mit fixiertem medizinischen Gerät, eine Ansicht der Folienschlauchabdeckung mit teilweise eingeführtem medizinischen Gerät,
- Figur 5: eine zweite Ansicht der Folienschlauchabdeckung mit teilweise eingeführtem medizinischen Gerät,
- Figur 6: eine dritte Ansicht der Folienschlauchabdeckung mit teilweise eingeführtem medizinischen Gerät,
- Figur 7: eine Ansicht der Folienschlauchabdeckung mit vollständig eingeführtem medizinischen Gerät,
- Figur 8: verschiedene Formen der Klebefolie.

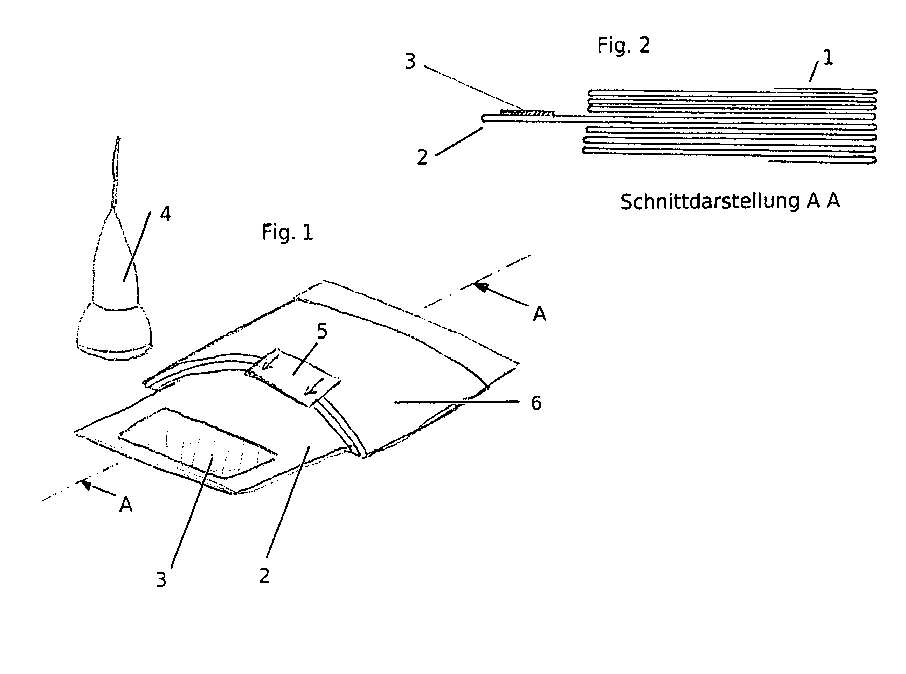

In Figur 1 ist eine Folienschlauchabdeckung gezeigt, die mit einer Steckfaltung 1 (Figur 2) verkürzt ist. An dem einen Ende 2 der Folienschlauchabdeckung, das sich nicht in der Steckfaltung befindet, ist eine Klebefläche in Form einer zweiseitig klebenden Klebefolie 3 vorhanden, an der die Kontaktfläche eines medizinischen Gerätes 4 fixiert wird. Die Steckfaltung kann sich auch in einer Kartoneinführhilfe 6 befinden. Die Öffnung der Steckfaltung wird über ein Pfeiletikett 5 gekennzeichnet. Dieses ist notwendig, um die Folienschlauchabdeckung beim Einführen des medizinischen Gerätes zu halten.

Figur 2 zeigt eine Schnittdarstellung zwischen den Punkten A - A der Figur 1. Die Folienschlauchabdeckung ist, wie hier zu sehen ist, durch eine Steckfaltung 1 verkürzt. An dem Ende 2 ist auf der nicht sterilen Seite die Klebefolie 3 aufgebracht.

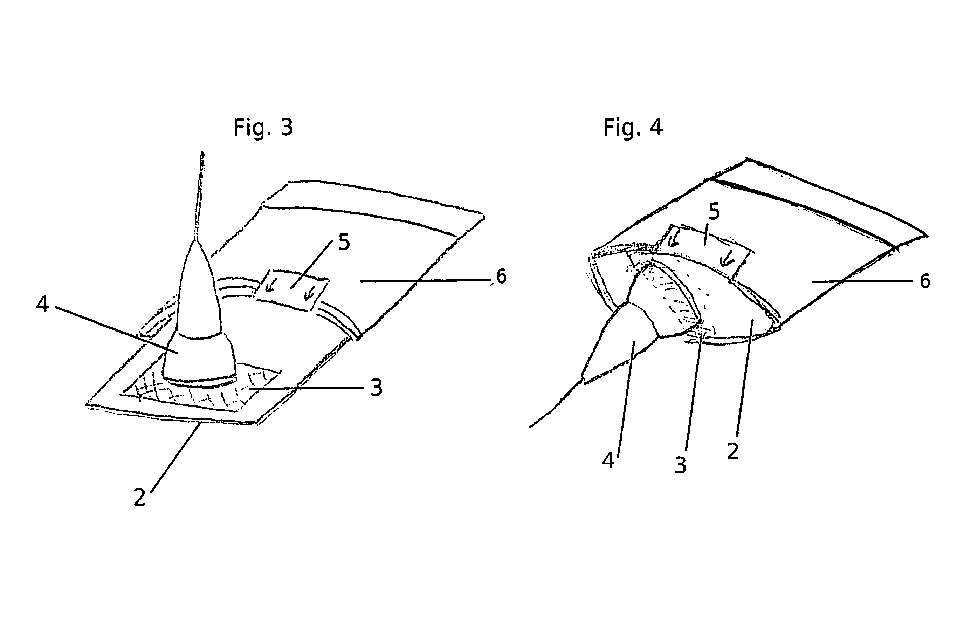

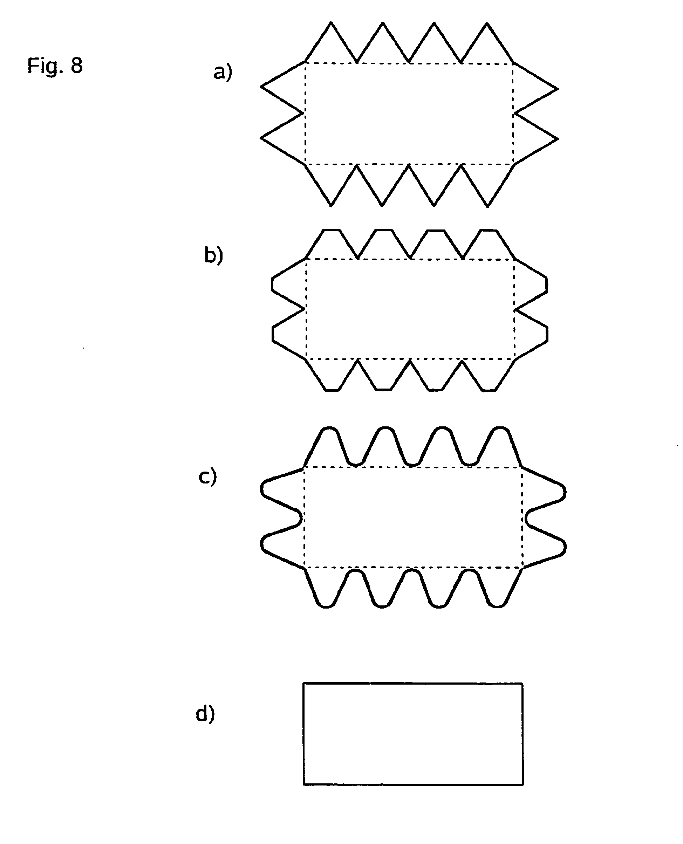

In Figur 3 wird das medizinische Gerät 4 an der Klebefolie 3 befestigt. Bei dem medizinischen Gerät handelt es sich vorzugsweise um ein Ultraschallgerät. Die Größe der Klebefolie 3 kann variieren, abhängig von dem verwendeten medizinischen Gerät. Bevorzugt ist, dass die Klebefolie 3 im Randbereich Einkerbungen aufweist. Bei medizinischen Geräten mit gekrümmter Kontaktfläche, beispielsweise Ultraschallköpfen, wird so vermieden, dass sich die Klebefolie 3 zusammen mit dem Folienschlauch an den Rändern der Kontaktfläche aufwölbt. Die Einkerbungen können verschiedene Formen aufweisen. In Figur 8 sind verschiedene Formen für Klebefolien gezeigt. So zeigt Figur 8a eine dreieckförmige, Figur 8b eine trapezförmige und Figur 8c eine wellenförmige Einkerbung. Jedoch sind auch andere Formen denkbar, solange beim Anliegen an einer gekrümmten Fläche keine Aufwölbung der Klebefolie 3 auftritt. Vorzugsweise ist die Klebefolie 3 dreieckig eingekerbt, da auf diese Weise die größtmögliche Krümmung auftreten kann. Jedoch ist auch eine im Wesentlichen rechteckige Klebefolie 3 denkbar, wie in Figur 8d gezeigt. Die Einkerbungen ermöglichen es, dass sich das unter den Einkerbungen liegende Folienschlauchmaterial leicht verformen kann, da an diesen Stellen keine Materialverstärkung durch die Klebefolie gegeben ist.

Wird das medizinische Gerät 4 nicht korrekt auf die Klebefolie 3 aufgesetzt oder bilden sich dabei Blasen oder Falten, und es ist wünschenswert, dies durch Nachspannen der Folie oder Ablösen und erneutes Aufsetzen des medizinischen Gerätes 4 zu korrigieren. Wird jedoch eine Klebefolie verwendet, die ein gleichmäßiges Klebeverhalten auf beiden Seiten aufweist, das heißt, dass die Verbindung mit dem medizinischen Gerät und die Verbindung mit dem Folienschlauch gleich stark sind, könnte dabei die Klebefolie 3 von dem Folienschlauch abgelöst werden statt von dem medizinischen Gerät 4. Daher wird bevorzugt eine Klebefolie 3 eingesetzt, die ein stark unterschiedliches Klebeverhalten aufweist.

Vorzugsweise weist die Klebefolie 3 ein Klebeverhalten von 60 zu 40 auf.

In Figur 4 ist zu sehen, dass das medizinische Gerät 4, das an der Klebefolie 3 befestigt ist, in die Steckfaltung 1 hineingeschoben wird. Durch die Fixierung an der Öffnung 2 der Steckfaltung mithilfe der Klebefolie 3 kann das medizinische Gerät 4 in die Steckfaltung hineingeschoben werden, ohne dass ein zusätzliches Festhalten der Folienschlauchabdeckung an dem medizinischen Gerät nötig ist. Wenn sich das medizinische Gerät vollständig in der Steckfaltung befindet, kann es durch die Haftung an der Klebefolie nicht mehr verrutschen. Um ein Kabel des medizinischen Gerätes innerhalb der Folienschlauchabdeckung zu fixieren, können im Bereich der Folienschlauchabdeckung Klebestreifen vorhanden sein.

Im Folgenden wird die genaue Verwendung der Folienschlauchabdeckung beschrieben.

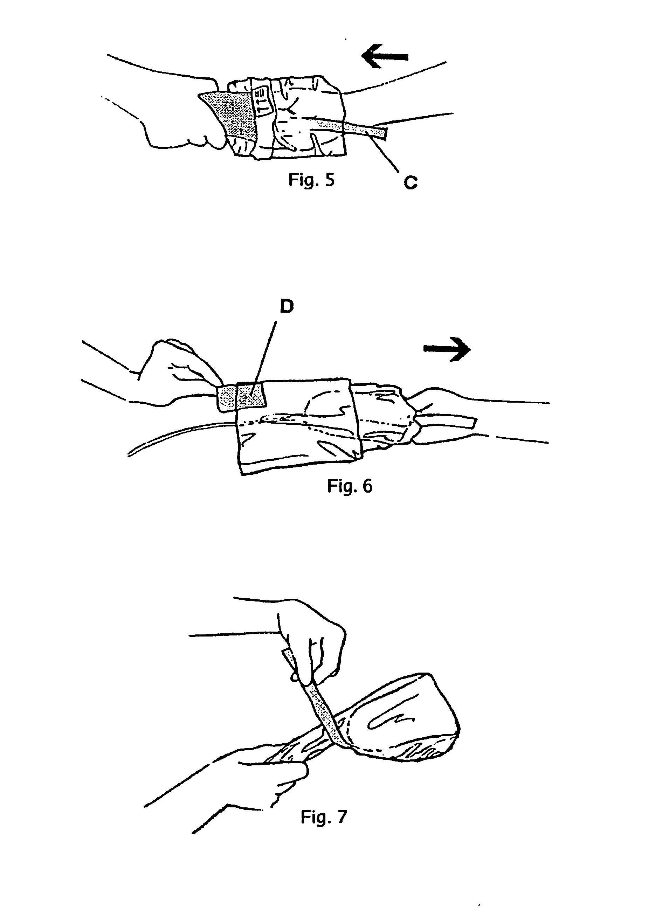

Zuerst wird das nicht sterile Gerät an der Klebefolie befestigt, nachdem eine nicht gezeigte Schutzfolie von dieser abgezogen wurde. Eine sterile Person greift an dem geschlossenen Endabschnitt in die Schlauchöffnung, wie es in Figur 5 zu sehen ist, und drückt die Klebefolie an das medizinische Gerät, das von einer nicht sterilen Person gehalten wird. Anschließend fasst die nicht sterile Person das offene Ende der Folienschlauchabdeckung an dem Einführetikett 5 und zieht den Schlauch entlang des Kabels des medizinischen Geräts (Figur 6). Die sterile Person hält das medizinische Gerät durch die Folienschlauchabdeckung fest. Das nicht sterile Gerät ist nun steril verpackt und eine Untersuchung kann steril durchgeführt werden. Das Kabel kann nun sowohl im sterilen als auch im nicht sterilen Bereich durch entsprechende Klebestreifen fixiert werden (Figur 7).

Obwohl im Vorstehenden eine getrennte Klebefolie 3 beschrieben wurde, ist es in gleicher Weise möglich, beispielsweise durch Druckverfahren, Haftklebematerial mit den in Figur 8 gezeigten Umrissen direkt auf den Folienschlauch aufzubringen und durch eine Schutzfolie abzudecken.

## Patentansprüche

1. Folienschlauchabdeckung für ein medizinisches Ultraschallgerät, mit einem durch eine Steckfaltung (1) verkürzten Folienschlauch, der einen offenen und einen geschlossenen Endabschnitt und eine sterile und eine nicht sterile Seite aufweist, **dadurch gekennzeichnet, dass** die Folienschlauchabdeckung auf einer Außenseite des geschlossenen Endabschnittes (2) auf der nicht sterilen Seite eine Klebefolie(3) zur Befestigung des Ultraschallgerätes (4) aufweist, derart, dass das Ultraschallgerät mit seiner Schallfläche an der Klebefolie (3) befestigt wird, die Klebefolie (3) als Schallübertragungsmedium dient und die Haftkraft der Klebefolie (3) auf der Folienschlauchseite größer als die Haftkraft der Klebefolie (3) auf der Ultraschallgeräteseite ist.

2. Folienschlauchabdeckung nach Anspruch 1, bei der das Verhältnis der Haftkraft der Klebefolie (3) auf der Folienschlauchseite gegenüber der Haftkraft der Klebefolie auf der Ultraschallgeräteseite ein Verhältnis von 60 zu 40 ist.

3. Folienschlauchabdeckung nach Anspruch 1 oder 2, **dadurch gekennzeichnet, dass** die Klebefolie (3) zumindest in einem Teil ihres Randbereichs Einkerbungen aufweist.

4. Folienschlauchabdeckung nach Anspruch 3, **dadurch gekennzeichnet, dass** die Einkerbungen dreieckförmig sind.

5. Folienschlauchabdeckung nach Anspruch 3, **dadurch gekennzeichnet, dass** die Einkerbungen trapezförmig sind.

6. Folienschlauchabdeckung nach Anspruch 3, **dadurch gekennzeichnet, dass** die Einkerbungen wellenförmig sind.

7. Folienschlauchabdeckung nach einem der vorhergehenden Ansprüche, **dadurch gekennzeichnet, dass** die Klebefolie (3) eine rechteckige Form aufweist.

8. Folienschlauchabdeckung nach einem der vorhergehenden Ansprüche, bei der die Klebefolie durch direkt auf den Folienschlauch aufgebrachtes Haftklebematerial gebildet ist.

9. Folienschlauchabdeckung nach einem der vorhergehenden Ansprüche, bei der die Folienschlauchabdeckung eine Öffnungsvorrichtung (5) aufweist, an der der offene Endabschnitt des Folienschlauches befestigt ist.

10. Folienschlauchabdeckung nach einem der Ansprüche 1 bis 9, bei der der Folienschlauch durch eine Zick-Zack-Faltung verkürzt ist.

11. Folienschlauchabdeckung nach Anspruch 9 bei der die Einführöffnung des Folienschlauches durch eine Kartonhülse (6) verstärkt ist.

12. Folienschlauchabdeckung nach Anspruch 8, bei der die Einführöffnung des Folienschlauches durch einen Ring verstärkt ist.

## Claims

1. A tubular film cover for a medical ultrasonic device comprising a film tube which is reduced in length by a collapsible folding (1) and which has an open and a closed end portion and a sterile and a non-sterile side, **characterised in that** the tubular film cover on an outside of the closed end portion (2) on the non-sterile side has an adhesive film (3) for fixing the ultrasonic device (4), in such a way that the ultrasonic device is fixed with its sound surface to the adhesive film (3), the adhesive film (3) serves as a sound transmission medium and the adhesive force of the adhesive film (3) on the tubular film side is greater than the adhesive force of the adhesive film (3) on the ultrasonic device side.

2. A tubular film cover according to claim 1 wherein the ratio of the adhesive force of the adhesive film (3) on the tubular film side in relation to the adhesive force of the adhesive film on the ultrasonic device side is a ratio of 60 to 40.

3. A tubular film cover according to claim 1 or claim 2 **characterised in that** the adhesive film (3) has notches at least in a part of its edge region.

4. A tubular film cover according to claim 3 **characterised in that** the notches are triangular.

5. A tubular film cover according to claim 3 **characterised in that** the notches are trapezoidal.

6. A tubular film cover according to claim 3 **characterised in that** the notches are wave-shaped.

7. A tubular film cover according to one of the preceding claims **characterised in that** the adhesive film (3) is of a rectangular shape.

8. A tubular film cover according to one of the preceding claims wherein the adhesive film is formed by contact adhesive material applied directly to the tubular film.

9. A tubular film cover according to one of the preceding claims wherein the tubular film cover has an opening device (5) to which the open end portion of the tubular film is fixed.

10. A tubular film cover according to one of claims 1 to 9 wherein the tubular film is reduced in length by a zig-zag folding.

11. A tubular film cover according to claim 9 wherein the introduction opening of the tubular film is reinforced by a cardboard sleeve (6).

12. A tubular film cover according to claim 8 wherein the introduction opening of the tubular film is reinforced by a ring.

## Revendications

1. Recouvrement en film tubulaire pour un appareil médical à ultrasons, avec un film tubulaire raccourci par un pliage télescopique (1), qui présente une portion d'extrémité ouverte et une portion d'extrémité fermée ainsi qu'un côté stérile et un côté
non stérile, **caractérisé en ce que** le recouvrement en film tubulaire présente, d'un côté extérieur de la portion d'extrémité fermée (2) du côté non stérile, une feuille adhésive (3) pour la fixation de l'appareil médical (4) de façon que l'appareil à ultrasons soit fixé à la feuille adhésive (3) par sa surface acoustique, la feuille adhésive (3) servant de milieu de transmission sonore et l'adhérence de la feuille adhésive (3) du côté du film tubulaire étant plus grande que l'adhérence de la feuille adhésive (3) du côté de l'appareil à ultrasons.

2. Recouvrement en film tubulaire selon la revendication 1, dans lequel le rapport entre l'adhérence de la feuille adhésive (3) du côté du film tubulaire et l'adhérence de la feuille adhésive du côté de l'appareil à ultrasons est de 60 sur 40.

3. Recouvrement en film tubulaire selon la revendication 1 ou 2, **caractérisé en ce que** la feuille adhésive (3) présente des encoches au moins dans une partie de sa région de bord.

4. Recouvrement en film tubulaire selon la revendication 3, **caractérisé en ce que** les encoches sont triangulaires.

5. Recouvrement en film tubulaire selon la revendication 3, **caractérisé en ce que** les encoches sont trapézoïdales.

6. Recouvrement en film tubulaire selon la revendication 3, **caractérisé en ce que** les encoches sont ondulées.

7. Recouvrement en film tubulaire selon une des revendications précédentes, **caractérisé en ce que** la feuille adhésive (3) présente une forme rectangulaire.

8. Recouvrement en film tubulaire selon une des revendications précédentes, dans lequel la feuille adhésive est formée par un matériau auto-adhésif appliqué directement sur le film tubulaire.

9. Recouvrement en film tubulaire selon une des revendications précédentes, dans lequel le recouvrement en film tubulaire présente un dispositif d'ouverture (5) auquel la portion d'extrémité ouverte du film tubulaire est fixée.

10. Recouvrement en film tubulaire selon une des revendications 1 à 9, dans lequel le film tubulaire est raccourci par un pli en zigzag.

11. Recouvrement en film tubulaire selon la revendication 9, dans lequel l'ouverture d'introduction du film tubulaire est renforcée par un manchon en carton (6).

12. Recouvrement en film tubulaire selon la revendication 8, dans lequel l'ouverture d'introduction du film tubulaire est renforcée par un anneau.
